(19) Europäisches Patentamt
European Patent Office
Office européen des brevets

(11) Publication number: 0 093 467 B1

(12) EUROPEAN PATENT SPECIFICATION

(45) Date of publication of patent specification: 02.07.86

(51) Int. Cl.[4]: C 07 D 209/44, B 01 J 31/22, B 01 J 31/24

(21) Application number: 83200546.6

(22) Date of filing: 18.04.83

# (54) Process for preparing 1,1,3,3-tetramethyl-2,3-dihydro-1-H-isoindoles substituted in position 5 and/or 6.

(30) Priority: 22.04.82 IT 2087482

(43) Date of publication of application:
09.11.83 Bulletin 83/45

(45) Publication of the grant of the patent:
02.07.86 Bulletin 86/27

(84) Designated Contracting States:
BE CH DE FR GB LI LU NL SE

(56) References cited:
DE-A-3 020 298

Chemical Abstracts vol. 62, no. 3, 1 February 1965, Columbus, Ohio, USA H. TOENJES et al. "Isoindolines. II. Synthesis of 1,1,3,3-tetrasubstituted isoindolines", column 2, Abstract no. 2754b

JOURNAL OF THE AMERICAN CHEMICAL SOCIETY, vol. 102, no. 16, 30 July 1980 R.L. FUNK et al. "Cooligomerizations of 3-substituted 1,5-hexadiynes with bis (trimethylsilyl) acetylene catalyzed by cobalt. A general synthesis of tricyclic ring systems from acyclic precursors", pages 5245-5253

(73) Proprietor: ENICHEM ANIC S.p.A.
Via Ruggero Settimo 55
I-90139 Palermo (IT)

(72) Inventor: Chiusoli, Gian Paolo
Via Solferino, 18 bis
I-43100 Parma (IT)
Inventor: Pallini, Luciano
Via V. Bottego, 3
I-43045 Fornovo di Taro, Parma (IT)
Inventor: Terenghi, Giuliana
Via C. Collodi, 18
I-42024 Castelnovo di Sotto Regg. Emilia (IT)

(74) Representative: Roggero, Sergio et al
Ing. Barzanò & Zanardo Milano S.p.A. Via Borgonuovo 10
I-20121 Milano (IT)

(56) References cited:
Chemical Abstracts vol. 63, no. 7, 27 September 1965, Columbus, Ohio, USA G.F. HENNION et al. "Sterically crowded amines. IV. Secondary and tertiary bispropargylic amines and their hydrogenation products", column 8180c-d

Journal Prakt. Chem. 26 (3-4) 218-24 (1964)

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 093 467 B1

## Description

This invention relates to a process for preparing substituted 1,1,3,3-tetramethyl-2,3-dihydro-1-H-isoindoles having the general formula:

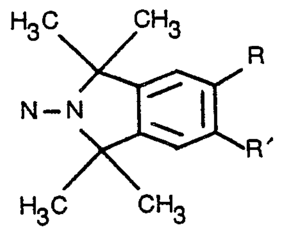

wherein R is an aminoisopropyl or a phenyl group, and R′ is hydrogen or a phenyl group, characterized by comprising the step of interacting di-tert.propargylamine with an acetylenic compound having the formula R—C≡C—R′, in the presence of a catalyst selected from the group consisting of a zerovalent nickel complex $NiL_n$ wherein L is a phosphine ligand and *n* is 2, 3 or 4, nickel Raney treated with a slight excess of thiourea and a NiX halide, wherein X is Cl, Br or I, reduced with Zn, Fe or Mn and thiourea, the interaction being carried out at a temperature comprised between 0°C and 100°C.

The reaction can be conducted either in the absence of solvents or in ether or nitrile solvents having a boiling point less than 150°C. Nickel-based catalysts are known to induce cyclotrimerisation in acetylenic compounds, with the formation of aromatic rings, but their catalytic activity in combining monoacetylenic with diacetylenic molecules was not known.

J.A.C.S. 102, page 5245 (1980) gives information regarding the activity, in selectively forming compounds deriving from a diacetylenic molecule and a monoacetylenic molecule, of certain cobalt complexes which are not easily accessible, are of high cost, and are of considerable toxicity.

It has now been found, and constitutes the subject matter of the present invention, that zerovalent complexes such as $Ni[C_6H_5—P—(O\text{-}i\text{-}propyl)_2]_4$ or $Ni[P—(O\text{-}isopropyl)_3]_4$, or Raney nickel treated with a slight excess of thiourea over that necessary for developing its active hydrogen, selectively catalyse the reaction between di-tert-propargylamine and monoacetylenic compounds to give isoindole derivatives with high yields, in accordance with the following scheme:

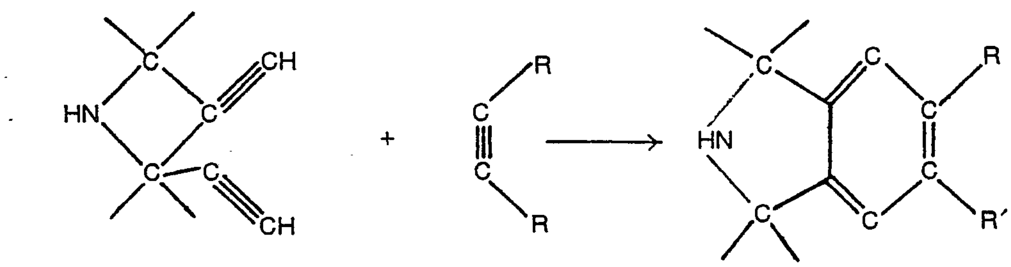

The reaction between the two molecules takes place very easily, it being necessary only to mix the reagents and catalyst together to obtain the desired result.

The diacetylenic amine and the monoacetylenic compound are introduced in a ratio which is equal to or less than 1:1 according to the acetylenic compound used. Generally, the preferred ratio is between 1:3 and 1:6.

The products obtained can be separated from the reaction mixture by conventional methods, preferably by direct distillation.

The products obtained are new chemical products which can be used as intermediates in organic synthesis for producing isoindole derivatives in all fields of application. The groups R and R′ can serve for functionalisation with suitable groups for further reaction with various substrates and supports.

Some illustrative examples are given hereinafter in order to further clarify the present invention.

### Example 1

1 g (6.71 mmoles) of di-tert-propargylamine (DTPA) and 4 ml of 3-methyl-3-amino-1-butyne (BAM) are fed into a 25 ml flask under an inert atmosphere. 140 mg (0.145 mmoles) of $Ni(PhP(OiPr)_2)_4$ are then added. As the reaction is very exothermic and could therefore proceed rather violently, the catalyst is added with caution, the reaction mixture being cooled with a water and ice bath. The low temperature is maintained until the solution has assumed a dark brown colour (about one hour), after which it is heated by an oil bath to 75—80°C for 4 hours.

It is allowed to cool to ambient temperature, and is hydrolysed, extracting with ethyl acetate.

The organic extracts are dried over anhydrous $Na_2SO_4$, and are subjected to gas chromatography analysis.

The gas chromatography yield of the product

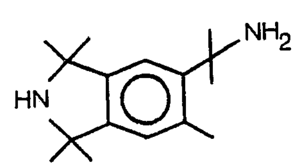

is 82% with respect to the fed DTPA, and the catalytic efficiency is 38 Moles of product per mole of catalyst.

The DTPA conversion is 100%.

The product, distilled in a bulb furnace (105—110°C, 2—2.5 mmHg) after acid-basic extraction of the reaction mixture, has a melting point of 63—65°C.

Example 2

1 g (6.71 mmoles) of DTPA and 4 ml of BAM are reacted under the same conditions as Example 1 in the presence of 138 mg (0.155 mmoles) of $Ni(P(OiPr)_3)_4$.

After 4 hours at 75—80°C, the reaction mixture is treated as in Example 1.

The gas chromatography yield of the product

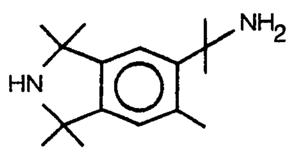

is 77% with respect to the fed DTPA, and the catalytic efficiency is 33 moles of product per mole of catalyst.

The DTPA conversion is 100%.

Example 3

0.906 g (6.08 mmoles) of DTPA, 3.8 ml of BAM, and, under agitation, 170 mg (0.131 mmoles) of $Ni(P(OPh)_3)_4$ are fed under an inert atmosphere into a 25 ml flask.

Initially heterogeneous, the mixture is heated under agitation to 75—80°C by means of an oil bath for 3 days. It is then allowed to cool to ambient temperature, hydrolysed, extracted with ethyl acetate, and subjected to gas chromatography analysis.

The gas chromatography yield of the product

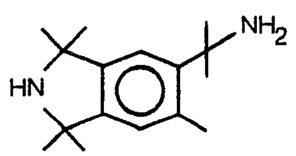

is 30% with respect to the fed DTPA, and the catalytic efficiency is 14 moles of product per mole of catalyst.

The DTPA conversion is 40%.

Analogous results are obtained by using $Ni(COD)_2$ or $Ni(PPh_3)_3$ in place of $Ni(P(OPh)_3)_4$.

Example 4

0.45 ml of Raney nickel suspended in water are fed under an inert atmosphere into a 25 ml flask. Washing is carried out, and the solvent replaced with ethanol. 80 mg of thiourea are then added, followed by a further 80 mg after the ethanol has been replaced with acetonitrile (about 3 ml). When gas ceases to be evolved, 0.500 g (3.35 mmoles) of DTPA and 2.5 ml of BAM are added (again under agitation), and the mixture heated to 75—80°C by means of an oil bath for 5 days. It is allowed to cool, filtered, and the solid residue washed repeatedly with acetonitrile. The filtrate is hydrolysed and extracted with ethyl acetate, and the organic extract, after drying over anhydrous $Na_2SO_4$, is subjected to gas chromatography analysis.

The gas chromatography yield of the product

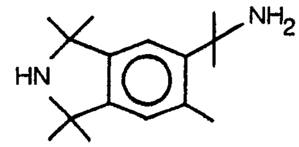

is 65% with respect to the fed DTPA. The DTPA conversion is 100%.

The catalyst, after recovering from the reaction mixture and taking-up in acetonitrile, has the same catalytic activity when again reacted under the same conditions.

Example 5

0.604 g (4.05 mmoles) of DTPA and 3.6 ml (32.8 mmoles) of phenyl acetylene in 7 ml of tetrahydrofuran (THF) are reacted together in the presence of 94 mg (0.098 mmoles) of $Ni(Ph\text{-}P(OiPr)_2)_4$ under the same conditions as Example 1.

After 20 hours at ambient temperature, the reaction mixture is treated as in Example 1.

The gas chromatography yield is the product

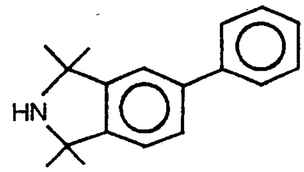

is 42% with respect to the fed DTPA, and the catalytic efficiency is 17 moles of product per mole of catalyst.

The DTPA conversion is 100%, the balance being converted almost exclusively into the product of addition of two molecules of phenylacetylene.

After acid-basic extraction of the reaction mixture and distilling in a bulb furnace (118—123°C/ 0.4—0.5 mmHg), the product has a melting point of 53—54°C.

Example 6

0.606 g (4.06 mmoles) of DTPA and 0.904 g (5.08 mmoles) of diphenyl acetylene dissolved in 7 ml of THF are reacted together in the presence of 82 mg (0.085 mmoles) of $Ni(PhP(OiPr)_2)_4$ under the same conditions as Example 1.

After 4 hours at 80°C, the reaction mixture is treated as in Example 1. The gas chromatography yield of the product

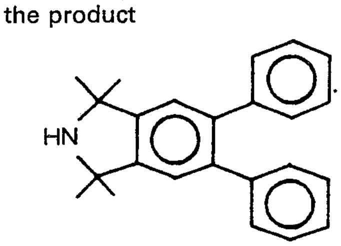

is 63% with respect to the fed DTPA, and the

catalytic efficiency is 30 moles of product per mole of catalyst.

The DTPA conversion is 100%.

The product is separated and purified as follows: the reaction mixture is hydrolysed and extracted with ethyl ether or ethyl acetate. The organic extract is treated with dilute $H_2SO_4$ (about 10%). A white solid separates at the interface, and is insoluble in both phases. It is filtered-off, washed with ethyl ether and crystallised from methanol or a mixture of ethanol and chlorobenzene (M.P. 215—216°C).

Example 7

0.355 g (2.38 mmoles) of DTPA and 2 ml of BAM are reacted together in the presence of 30 mg (0.23 mmoles) of $NiCl_2$, 73 mg of Zn dust and 50 mg of thiourea under the same reaction conditions as Example 1. After 3 days at 75°C, the reaction mixture is treated as in Example 1. The gas chromatography yield of the product

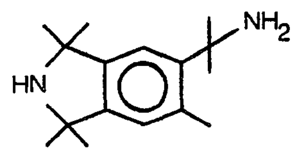

is 17% with respect to the fed DTPA, and the catalytic efficiency is about 2 moles of product per mole of $NiCl_2$ used.

**Claim**

A process for preparing substituted 1,1,3,3-tetramethyl-2,3-dihydro-1-H-isoindoles having the general formula:

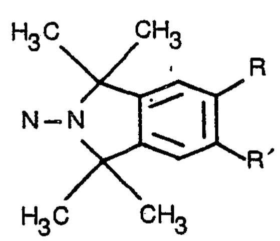

wherein R is an aminoisopropyl or a phenyl group, and R′ is hydrogen or a phenyl group, characterized by comprising the step of interacting di-tert.propargylamine with an acetylenic compound having the formula R—C≡C—R′, in the presence of a catalyst selected from the group consisting of:

a zerovalent nickel complex $NiL_n$ wherein L is a phosphine ligand and *n* is 2, 3 or 4;

nickel Raney treated with a slight excess of thiourea, and

a $NiX_2$ halide, wherein X is Cl, Br or I, reduced with Zn, Fe, or Mn and thiourea, the interaction being carried out at a temperature comprised between 0°C and 100°C.

**Patentanspruch**

Verfahren zur Herstellung von substituierten 1,1,3,3 - Tetramethyl - 2,3 - dihydro - 1 - H - isoindolen der allgemeinen Formel

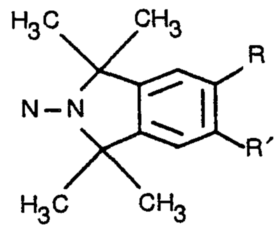

worin R eine Aminoisopropyl- oder eine Phenylgruppe ist und R′ Wasserstoff oder eine Phenylgruppe darstellt, dadurch gekennzeichnet, daß der Schritt der Umsetzung von di-tert.Propargylamin mit einer acetylenischen Verbindung der Formel R—C≡C—R′ durchgeführt wird in Gegenwart eines Katalysators ausgewählt aus der Gruppe bestehend aus:

einem nullwertigen Nickelkomplex $NiL_n$, worin L ein Phosphinligand ist und n 2, 3 oder 4 ist;

Raney-Nickel behandelt mit einem leichten Überschuß von Thioharnstoff und

einem $NiX_2$-Halogenid, worin X Chlor, Brom oder Jod ist, reduziert mit Zn, Fe oder Mn und Thioharnstoff, wobei diese Umsetzung bei einer Temperatur zwischen 0°C und 100°C durchgeführt wird.

**Revendication**

Procédé de préparation de 1,1,3,3-tetraméthyl-2,3-dihydro-1-H-isoindoles substitués, de formule générale:

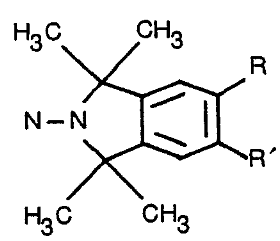

dans laquelle R est un groupe amino-isopropyl ou phényl, et R′ est un atome d'hydrogène ou un groupe phényle, caractérisé en ce qu'il comprend l'étape d'interaction de la di-tert.propargyl-amine avec un composé acétylénique de formule R—C≡C—R′, en présence d'un catalyseur choisi parmi le groupe constitué de:

un complexe du nickel zérovalent $NiL_n$ dans lequel L est un ligand phosphine et *n* vaut 2, 3, ou 4;

du nickel de Raney traité avec un faible excès de thiourée, et

un halogénure $NiX_2$, dans lequel X est Cl, Br, ou I, réduit avec Zn, Fe, ou Mn et de la thiourée, cette interaction étant effectuée à une température comprise entre 0 et 100°C.